# EUROPÄISCHE PATENTSCHRIFT

(11) **EP 2 643 025 B1**
(45) Veröffentlichungstag und Bekanntmachung des Hinweises auf die Patenterteilung: **05.11.2014**
(21) Anmeldenummer: 11757552.2
(22) Anmeldetag: 13.09.2011
(51) Int. Cl.: A61L 2/10, B67B 3/00

(54) **VORRICHTUNG ZUM STERILISIEREN VON VERSCHLÜSSEN FÜR BEHÄLTER**
DEVICE FOR STERILIZING CLOSURES FOR CONTAINERS
DISPOSITIF POUR STÉRILISER DES FERMETURES DE CONTENANTS

(30) Priorität: 24.11.2010 DE 102010052207
(43) Veröffentlichungstag der Anmeldung: 02.10.2013
(73) Patentinhaber: KHS GmbH, 44143 Dortmund (DE)
(72) Erfinder: DRENGUIS, Alfred, 21039 Börnsen (DE)
(86) Internationale Anmeldenummer: PCT/EP2011/004595
(87) Internationale Veröffentlichungsnummer: WO 2012/069101

(56) Entgegenhaltungen:
- WO-A2-2006/106363
- GB-A- 1 093 751
- US-A- 2 175 682

## Beschreibung

Die Erfindung bezieht sich auf eine Vorrichtung zum Entkeimen bzw. Sterilisieren von Verschlüssen gemäß Oberbegriff Patentanspruch 1.

Beispielsweise bei Anlagen der Getränkeindustrie ist es üblich und bekannt, die zum Verschließen von gefüllten Flaschen oder anderen Behältern verwendete Verschlüsse, insbesondere auch kappenartige Verschlüsse, wie Schraubverschlüsse, Flachkappen, Kronkorken usw. vor ihrer Verwendung, d.h. vor dem Aufbringen auf den jeweiligen Behälter zu sterilisieren bzw, zu entkeimen.

Für dieses Sterilisieren eignet sich u.a. auch UV-Strahlung, insbesondere UV-C-Strahlung, mit der die Verschlüsse an ihren Außen- und Innenfläche während einer ausreichenden Behandlungsdauer, beispielsweise während einer Behandlungszeit von etwa 120 Sekunden beaufschlagt werden.

UV-Strahlungsquellen im Sinne der Erfindung sind insbesondere alle bekannten UV-Strahlungsquellen oder UV-Lampen, so u.a. quecksilberdotierte Hochdruck- und Niederdrucklampen bzw. Hochdruck- und Niederdruck-Gasentladungslampen, aber auch UV-Strahlung aussendende Quarz-Lampen, Deuterium-Lampen usw. Bevorzugt sind UV-Strahlungsquellen im Sinne der Erfindung UV-Licht aussendende Niederdruckgasentladungslampen und dabei vorzugsweise stabförmige UV-Licht aussende Niederdruck-Gasentladungslampen.

Aus der GB1,093,751 ist bereits eine Vorrichtung und ein Verfahren bekannt, bei welchem UV-transparente Behälter vor dem Füllen an lokalen UV-Strahlern vorbei transportiert werden. Dabei werden weiterhin Verschlusskappen stromabwärts des Füllers in Zuführkanälen einer feststehenden Verschließereinheit geführt, wobei diese durch ein UV-Strahlungsfeld geleitet werden.

Aufgabe der Erfindung ist es, eine Vorrichtung aufzuzeigen, die bei hoher Qualität der Sterilisation oder Entkeimungsrate auch eine hohe Leistung (Anzahl der sterilisierten bzw. entkeimten Verschlüsse je Zeiteinheit) reproduzierbar ermöglicht. Zur Lösung dieser Aufgabe ist eine Vorrichtung entsprechend dem Patentanspruch 1 ausgebildet.

Besondere Vorteile der erfindungsgemäßen Vorrichtung besehen u.a. darin, dass diese trotz hoher Leistung sehr kompakt und mit kleinem Bauraum oder Bauvolumen realisierbar ist, dass gleichbleibend, d.h. reproduzierbar hohe Entkeimungsraten bei der Sterilisation erreicht werden und dass die Entkeimung bzw. Sterilisation chemiefrei erfolgt, und zwar bei geringen Betriebskosten, die sich u.a. aus einem reduzierten Leistungsbedarf vor allem für den Betrieb der UV-Strahlungsquellen und aus der hohen Lebensdauer dieser Strahlungsquellen ergeben. Die erfindungsgemäße Vorrichtung eignet sich für Verschlüsse unterschiedlichster Art, insbesondere auch für kappenartige Verschlüsse, wie Schraubverschlüsse, Flachkappen, Kronkorken usw.

Bevorzugt ist bei der erfindungsgemäßen Vorrichtung der wenigstens eine Behandlungsraum mit einem leichten Überdruck eines sterilen gas- und/oder dampfförmigen Mediums, beispielsweise steriler Luft beaufschlagt, sodass ein Eindringen von Umgebungsluft und von mit dieser mitgeführten Keimen in den Behandlungsraum bzw. in die Behandlungszone wirksam vermieden ist, vor allem auch im Bereich von Verschlusseinlässen und Verschlussauslässen. Durch die UV-Strahlung im Behandlungsraum erzeugtes Ozon wirkt auf die Verschlüsse ebenfalls sterilisierend bzw. entkeimend und unterstützt damit die Sterilisier- und/oder Entkeimungswirkung der UV-Strahlung.

Die Erfindung wird im Folgenden anhand der Figuren an einem Ausführungsbeispiel näher erläutert. Es zeigen:

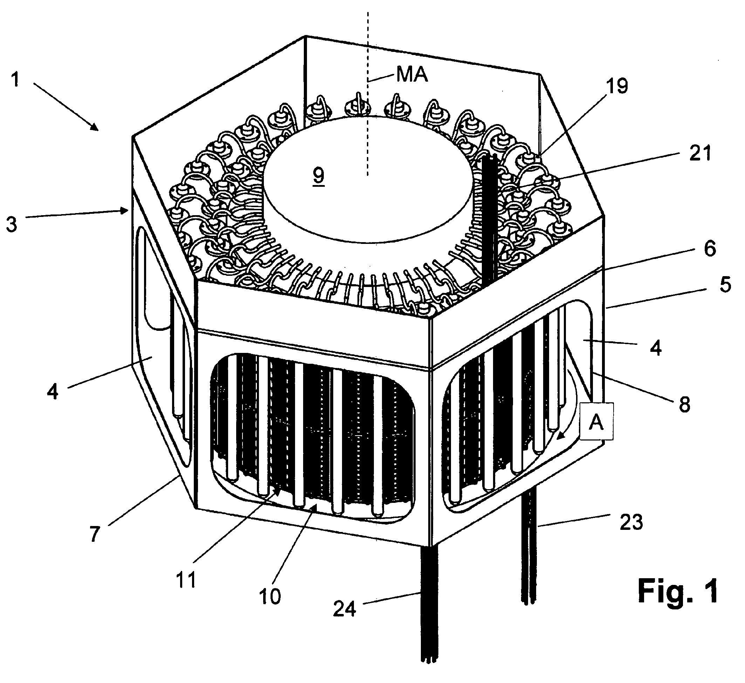

- Fig. 1: in perspektivischer Darstellung eine Vorrichtung gemäß der Erfindung zum Sterilisieren von Verschlüssen, die zum Verschließen von Flaschen oder dergleichen Behältern bestimmt sind;

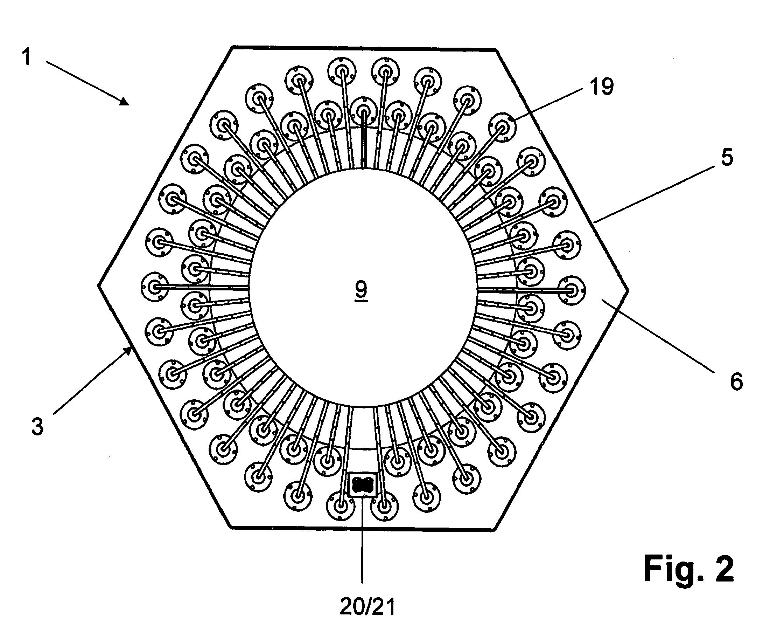

- Fig. 2: eine Draufsicht auf die Vorrichtung der Figur 1;

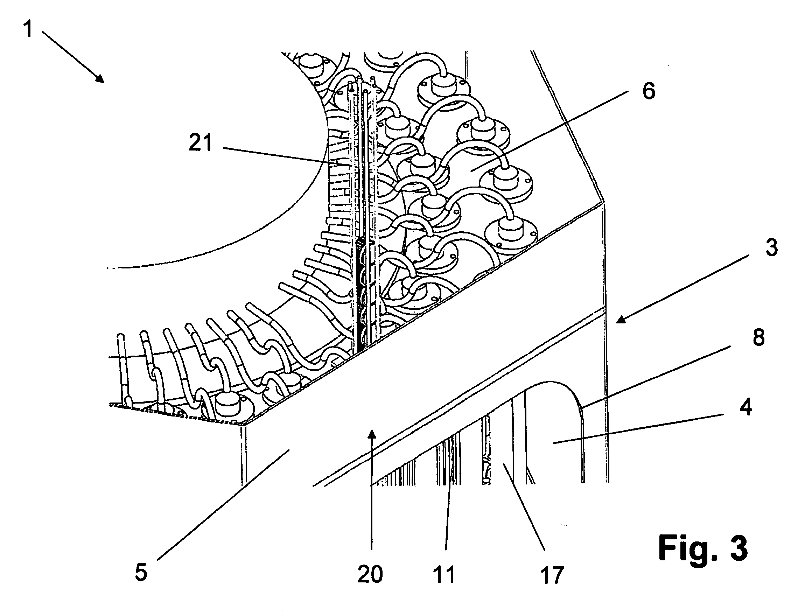

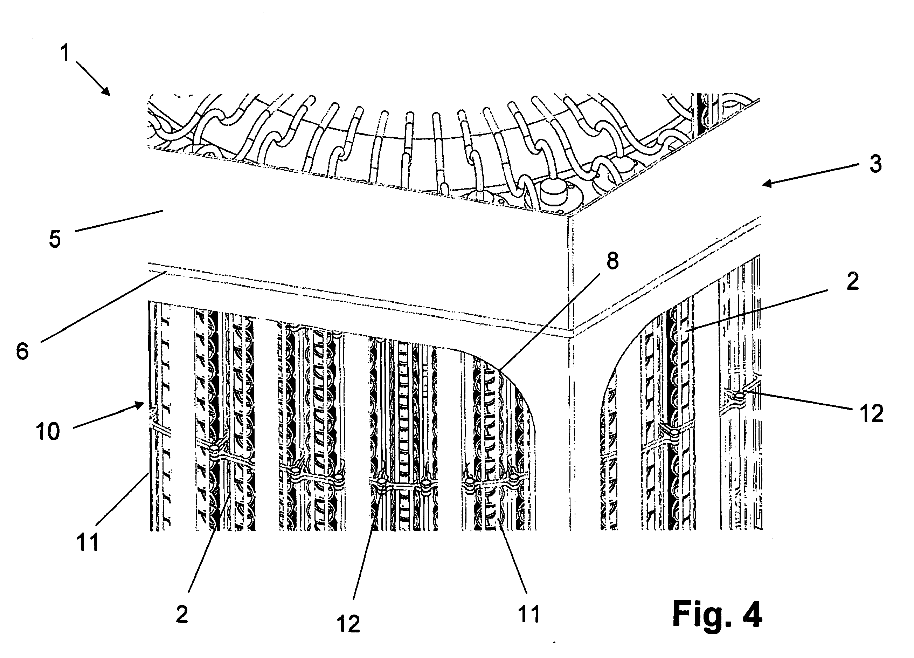

- Fig. 3 und 4: jeweils in perspektivischer Teildarstellung die Vorrichtung der Figur 1 in einer Blickrichtung von oben;

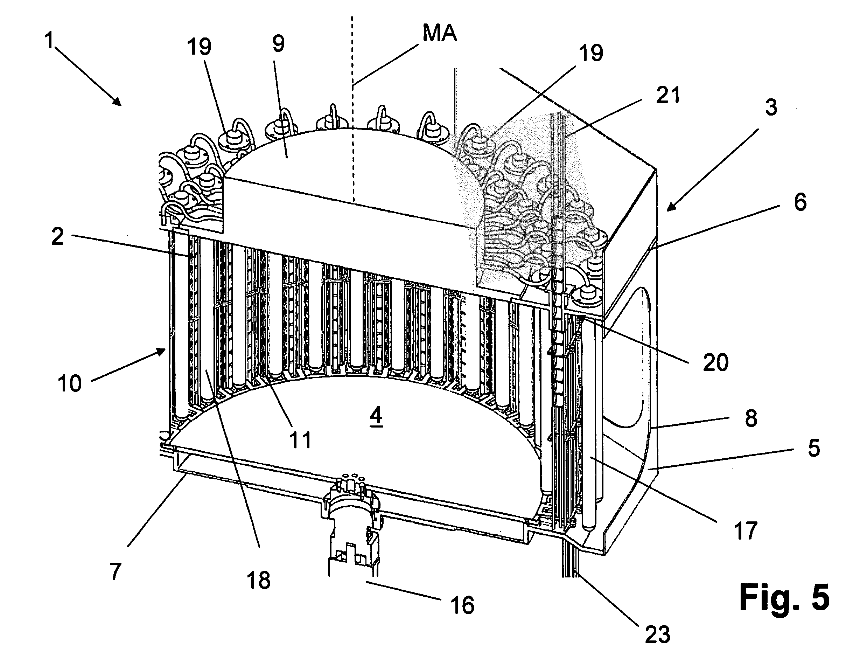

- Fig. 5: die Vorrichtung der Figur 1 in perspektivischer Darstellung sowie im Schnitt;

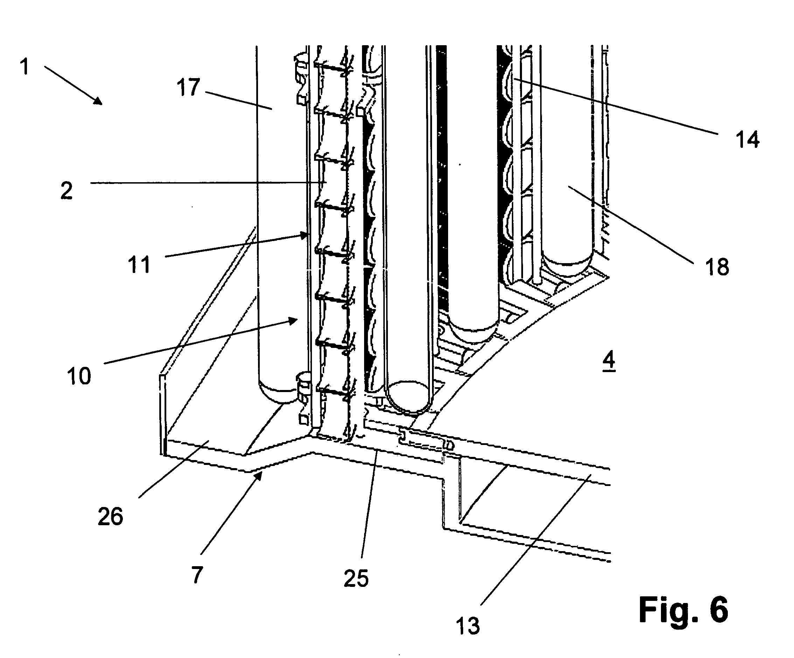

- Fig. 6: eine vergrößerte Teildarstellung der Figur 5;

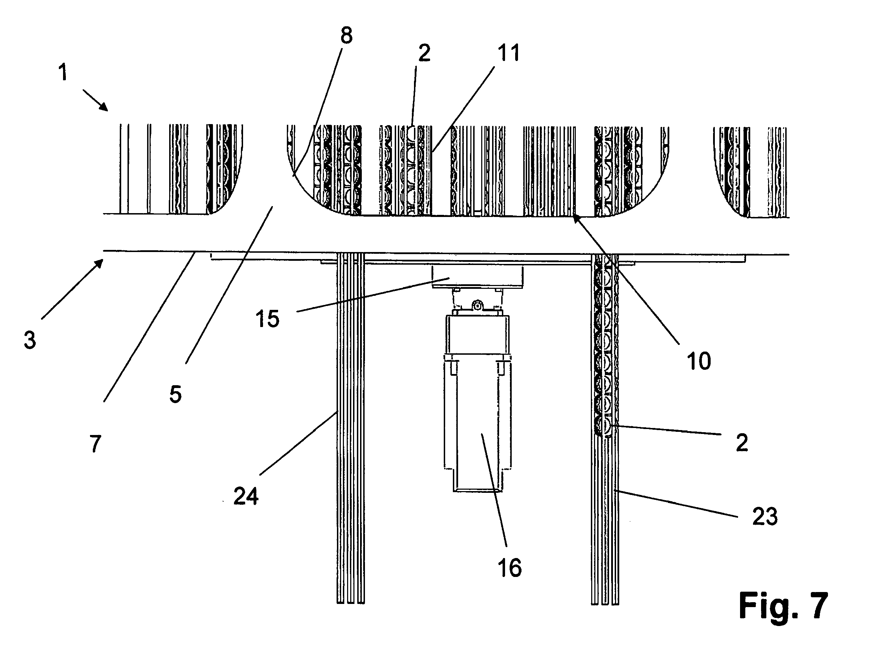

- Fig. 7: die Vorrichtung der Figur 1 in Seitenansicht und im Bereich einer unteren Verschlussabgabe bzw. Verschlussförderstrecke;
- Fig. 8: in vereinfachter Teildarstellung und in Seitenansicht zwei der an einem Rotor der Vorrichtung gebildeten Verschlussaufnahmen.

Die in den Figuren allgemein mit 1 bezeichnete Vorrichtung dient zum Sterilisieren oder Entkeimen von Verschlüssen 2, beispielsweise von Verschlüssen in Form von Kappen, Schraubkappen, Sportkappen, Flachkappen oder Kronenkorken usw., die zum Verschließen von nicht dargestellten Behältern, z.B. in Form von Flaschen verwendet werden. Die Vorrichtung 1 bildet hierfür bevorzugt ein einer Verschließmaschine zum Verschließen der Behälter vorgeschaltetes Aggregat, aus dem die sterilisierten Verschlüsse 2 unter sterilen bzw. keimfreien Bedingungen der Verschließmaschine zugeführt werden.

Die Vorrichtung umfasst u.a. ein Vorrichtungsgehäuse 3, welches bei der dargestellten Ausführungsform in Draufsicht eine eine vertikale Maschinen- oder Rotorachse MA umschließende polygonale Formgebung aufweist, d.h. sechseckförmig ausgebildet ist. Der Innenraum 4 des Gehäuses 3 ist mit Ausnahme von Ein- und Auslässen für die Verschlüsse 2 gegenüber der Umgebung dicht verschlossen, und zwar durch eine die Maschinenachse MA umschließende Umfangswand 5, durch eine obere bei der dargestellten Ausführungsform sechseckförmige Gehäusewand 6 und eine untere ebenfalls sechseckförmige Gehäusewand 7. In der Umfangswand 5 sind Inspektionsfenster 8 vorgesehen, die jeweils bevorzugt für UV-Licht undurchlässig oder für UV-Licht zumindest stark dämpfend ausgeführt, beispielsweise aus einem entsprechend ausgestatteten transparenten Kunststoff oder Glas, und/oder aber durch nicht dargestellte Klappen verschließbar sind. An der Oberseite des Gehäuses 3 bzw. im Bereich der dortigen Gehäusewand 6 ist ein über die Oberseite dieser Gehäusewand vorstehender Gehäuseteil 9 vorgesehen, über den u.a. der Innenraum 4 für Reparatur- und/oder Wartungszwecke zugänglich ist.

Im Innenraum 4 des Gehäuses 3 ist ein um die vertikale Maschinenachse MA umlaufend antreibbarer Rotor 10 aufgenommen. Bei der dargestellten Ausführungsform ist der Rotor 10 als kreiszylinderförmige, die Maschinenachse MA konzentrisch umschließende Hohltrommel mit einem Trommelmantel mit käfigartiger Struktur ausgeführt, die von einer Vielzahl von Verschlussaufnahmen 11 zur Aufnahme jeweils einer Vielzahl von Verschlüssen 2 gebildet ist. Die Verschlussaufnahmen 11, die mit ihrer Längserstreckung jeweils parallel oder im Wesentlichen parallel zur Maschinenachse MA orientiert und an ihrem oberen Ende zum Einbringen der Verschlüsse 2 und an ihrem unteren Ende zum Ausbringen der Verschlüsse 2 offen sind, bilden in ihrer Gesamtheit die käfig- oder gitterartige Struktur bzw. den käfigartigen, in sich stabilen Mantel des Rotors 10 und sind hierfür am Umfang des Rotors 10 in gleichmäßigen Winkel- und Teilsabständen um die Maschinenachse MA verteilt vorgesehen. Durch die Maschinenachse MA konzentrisch umschließende und in Richtung dieser Achse gegeneinander versetzte ringartige Halterungen 12 sind die Verschlussaufnahmen 11 miteinander zu der gitterartigen Struktur des Rotors 10 verbunden. Im Bereich ihrer unteren Enden sind die Verschlussaufnahmen 11 an einem achsgleich mit der Maschinenachse MA angeordneten kreisscheibenförmigen Tragelement 13 befestigt, und zwar derart, dass die Verschlussaufnahmen 11 jeweils über den Umfang des Tragelementes 13 vorstehen und die unteren, offenen Enden der Verschlussaufnahmen 11 durch das Tragelement 13 nicht abgedeckt sind, vielmehr im Bereich des Tragelementes 13 oder unterhalb dieses Tragelementes freiliegen.

Die Verschlussaufnahmen 11 sind ebenfalls gitterartig ausgeführt und bestehen jeweils aus mehreren voneinander beabstandeten und parallel zur Maschinenachse MA orientierten stangen- oder stabförmigen Verschlussführungsschienen 14, die zwischen sich einen Aufnahmeraum zur Aufnahme einer Vielzahl von Verschlüssen 2 bilden, und zwar derart, dass die Verschlüsse 2 in jeder Verschlussaufnahme 11 eine sich in einer Achsrichtung parallel zur Maschinenachse MA erstreckende einspurige Verschlussreihe oder -gruppe bildend und in der jeweiligen Verschlussaufnahme 11 möglichst weitgehend freiliegen, d.h. nur an einem geringen Teil ihrer Oberfläche von den Verschlussführungsschienen 14 abgedeckt sind. Weiterhin sind die Verschlussaufnahmen 11 bei der dargestellten Ausführungsform so ausgeführt, dass die Verschlüsse 2 in den Verschlussaufnahmen 11 in Bezug auf die Maschinenachse MA eine vorgegebene Orientierung aufweisen, und zwar in der Form, dass sie mit ihrer Verschluss- oder Kappenachse radial zur Maschinenachse MA und beispielsweise mit ihrer offenen Kappenseite radial nach außen orientiert sind.

Mit dem Tragelement 13 ist der Rotor 10 in einem Lager 15 an der Unterseite des Gehäuses 3 bzw. an der dortigen unteren Gehäusewand 7 um die Maschinenachse MA drehbar gelagert und mittels eines Antriebs 16 (Elektromotor mit Getriebe) um die Maschinenachse MA entsprechend dem Pfeil A getaktet bzw. schrittweise umlaufend antreibbar.

Mit dem Rotor 10 nicht umlaufend sind in dem Innenraum 4 des Gehäuses 3, d.h. in dem Behandlungsraum der Vorrichtung 1 UV-Lichtquellen verteilt vorgesehen, und zwar bei der dargestellten Ausführungsform in Form von stabförmigen UV-Lampen 17 und 18, die jeweils hängend an der oberen Gehäusewand 6 bzw. an dortigen Lampenfassungen 19 gehalten und mit ihren Achsen parallel zu Maschinenachse MA orientiert sind. Die äußeren UV-Lampen 17 sind dabei bezogen auf die Maschinenachse MA radial außerhalb der Bewegungsbahn der Verschlussaufnahmen 11 und die inneren UV-Lampen 18 bezogen auf die Maschinenachse MA radial innerhalb der Bewegungsbahn der Verschlussaufnahmen 11 bzw. der von diesen Verschlussaufnahmen 11 gebildeten käfigartigen Struktur vorgesehen, und zwar bei der dargestellten Ausführungsform derart auf Lücke versetzt, dass bezogen auf die Maschinenachse MA jeder inneren UV-Lampe 18 eine Lücke zwischen zwei äußeren UV-Lampen 17 radial gegenüber liegt. Die Anzahl der inneren UV-Lampen ist beispielsweise gleich oder annähernd gleich der Anzahl der Verschlussaufnahmen 11 am Rotor 10. Bevorzugt ist aber die Anzahl der äußeren UV-Lampen 17 und der inneren UV-Lampen 18 jeweils kleiner als die Anzahl der Verschlussaufnahmen 11. Durch die Anordnung der UV-Lampen 17 und 18, insbesondere auch durch die versetzte Anordnung dieser Lampen ist gewährleistet, dass die in den Verschlussaufnahmen 11 aufgenommenen Verschlüsse auf der UV-Behandlungsstrecke, die von dem Winkelbereich der Drehbewegung des Rotors 10 zwischen der Verschlussaufgabe 20 und der Verschlussabgabe 22 gebildet ist, durch ein möglichst gleichmäßiges UV-Strahlungsfeld bewegt werden. In dem domartigen Gehäuseteil 9 sind u.a. die für die Ansteuerung der UV-Lampen 17 und 18 notwendigen elektrischen Funktionselemente untergebracht, mit denen die UV-Lampen 17 und 18 bzw. deren Lampenfassungen 19 über elektrische Leitungen verbunden sind.

Die UV-Lampen 17 und 18, die bevorzugt identisch oder im Wesentlichen identisch ausgebildet sind, erstrecken sich jeweils mit ihrem unteren, freien Ende bis in die Nähe des Niveaus der Oberseite des scheibenförmigen Tragelementes 13 und sind dort geringfügig von der Oberseite bzw. dem Niveau der Oberseite des Tragelementes beabstandet. Grundsätzlich besteht die Möglichkeit, die äußeren UV-Lampen 17 mit einer etwas größeren Länge auszubilden, und zwar derart, dass diese Lampen mit ihrem unteren, freien Ende über die Ebene des Tragelementes 13 nach unten vorstehen und in einen rinnenartigen Abschnitt 26 hineinreichen, der im äußeren Bereich des Innenraumes 4 aus der unteren Gehäusewand 7 geformt ist und die Maschinenachse MA kreisringartig umschließt. Durch diese Anordnung und Ausbildung der äußeren UV-Lampen 17 ist besonders zuverlässig gewährleistet, dass auch die in den Verschlussaufnahmen 11 jeweils untersten Verschlüsse 2, aber auch die mit diesen Verschlüssen 2 in Berührung kommenden Bereiche, insbesondere auch die Fläche 25 intensiv mit UV-Strahlung behandelt bzw. beaufschlagt werden.

Im oberen Bereich des Innenraumes 4 bzw. des von diesen gebildeten Behandlungsraumes ist über der Bewegungsbahn der Verschlussaufnahmen 11 eine Verschlussaufgabe 20 vorgesehen, die von einem unteren Ende einer äußeren Verschlussförderstrecke 21 gebildet ist und der die zu sterilisierenden Verschlüsse 2 über diese Verschlussförderstrecke 21 zugeführt werden. In der Verschlussförderstrecke 21 weisen die Verschlüsse 2 bereits die ihrer Orientierung in den Verschlussaufnahmen 11 entsprechende Orientierung auf. Im Bereich der Unterseite des Gehäuses 3 ist unterhalb der Bewegungsbahn der Verschlussaufnahmen 11 eine Verschlussabgabe 22 vorgesehen, die im Wesentlichen von dem Einlass oder von einem oberen offenen Ende einer Verschlussförderstrecke 23 gebildet ist, welche durch die untere Gehäusewand 7 hindurch in den Innenraum 4 hineinreicht und mit ihrem offenen Ende unterhalb der Bewegungsbahn der Verschlussaufnahmen 11 des Rotors 10 angeordnet ist und dort die Verschlussabgabe 22 bildet. Über die Verschlussförderstrecke 23 werden die sterilisierten Verschlüsse 2 der nicht dargestellten Verschließmaschine keimfrei zugeführt. Zusätzlich zur Verschlussabgabe 22 und der Verschlussförderstrecke 23 ist eine von einer Verschlussführung 24 gebildete Verschlussabgabe unterhalb der Bewegungsbahn der Verschlussaufnahmen 11 vorgesehen, über die ein schnelles Leerfahren der Vorrichtung 1 bzw. des Rotors 10, d.h. ein beschleunigtes Entfernen der Verschlüsse 2 aus den Verschlussaufnahmen 11 möglich ist, und zwar beispielsweise bei Störungen in der Vorrichtung 1, in einer die Vorrichtung 1 aufweisenden Anlage, bei einem Formatwechsel, d.h. beim Umstellen von einer Verschlussart auf eine andere Verschlussart usw.

Die aus dem Gehäuse 3 herausgeführte Verschlussführung 24 endet im Innenraum 4 unterhalb der Bewegungsbahn der Verschlussaufnahmen 11. Durch entsprechende, nicht dargestellte Steuermittel ist der dortige, von der Verschlussführung 24 gebildete Auslass derart steuerbar, dass im normalen Betrieb der Vorrichtung 1 Verschlüsse 2 nicht in die Verschlussführung 24 gelangen, sondern lediglich beim beschleunigten Leerfahren der Vorrichtung 1 und/oder des Rotors 10.

Bei der dargestellten Ausführungsform sind die Verschlussförderstrecken 21 und 23 und die Verschlussführung 24 jeweils von mehreren, die Verschlüsse 2 zwischen sich aufnehmenden und führenden Führungsschienen gebildet. Weiterhin weisen bei der dargestellten Ausführungsform die Verschlussförderstrecken 21 und 23 sowie die Verschlussführung 24 zumindest in der Nähe der Vorrichtung 1 jeweils einen vertikalen Verlauf auf, sodass die Verschlüsse 2 in den Verschlussförderstrecken 21 und 23 sowie in der Verschlussführung 24 allein schon aufgrund ihrer Schwerkraft bewegt bzw. gefördert werden. Um allerdings ein erneutes Verkeimen der sterilisierten Verschlüsse 2 auf der Verschlussförderstrecke 23 zu vermeiden ist diese in einer nicht dargestellten Ummantelung oder Einhausung aufgenommen, die bevorzugt mit einem Überdruck eines sterilen gas- und/oder dampfförmigen Mediums, beispielsweise mit dem Überdruck steriler Luft beaufschlagt ist.

Die Verschlussaufgabe 20 und die Verschlussabgabe 22 sind bezogen auf die Maschinenachse MA in einem Winkelabstand, der einem Vielfachen des Teilungsabstandes der Verschlussaufnahmen 11 am Rotor 10 entspricht, so angeordnet, dass immer dann, wenn sich eine Verschlussaufnahme 11 mit ihrem oberen offenen Ende in der Stillstandsphase der Drehbewegung des Rotors 10 an der Verschlussaufgabe 20 befindet, sich eine andere Verschlussaufnahme 11 mit ihrem unteren offenen Ende an der Verschlussabgabe 22 befindet. Weiterhin ist die Verschlussabgabe 22 bezogen auf die Drehrichtung A des Rotors 10 um einen möglichst großen Winkelbetrag, beispielsweise um einen Winkelbetrag etwas kleiner als 360°, z.B. um einen Winkelbetrag von 330° oder von etwa 330° von der Verschlussaufgabe 20 beabstandet, um eine möglichst lange Behandlungsstrecke und damit auch bei hohen Leistungen der Vorrichtung 1, d.h. bei einer hohen Anzahl der je Zeiteinheit mit dieser Vorrichtung 1 sterilisierten Verschlüssen 2 bzw. bei einer entsprechend hohen Drehgeschwindigkeit des Rotors 10 eine möglichst lange Behandlungsdauer für das Entkeimen bzw. Sterilisieren der Verschlüsse 2 zu erreichen, und zwar bei reduzierten Durchmesser des Rotors 10 und kompakter Bauform der Vorrichtung 1 insgesamt.

Mit dem Antrieb 17 wird der Rotor 10 getaktet um die Maschinenachse MA angetrieben und zwar derart, dass der Rotor 10 in jeder Bewegungsphase der getakteten Drehbewegung einen dem Teilungsabstand zweier Verschlussaufnahmen 11 entsprechenden Drehschritt ausführt und sich in jeder Stillstandsphase der getakteten Drehbewegung eine leere Verschlussaufnahme 11 unterhalb der Verschlussaufgabe 20 befindet, über die dann aus der Verschlussförderstrecke 21 die Verschlussaufnahme 11 vollständig mit Verschlüssen 2 gefüllt wird. Mit der getakteten Drehbewegung des Rotors 10 werden die jeweils mit den Verschlüssen 2 gefüllten Verschlussaufnahmen 11 entlang der UV-Lampen der von den UV-Lampen 17 und 18 gebildeten und sich über den Winkelbereich zwischen der Verschlussaufgabe 20 und der Verschlussabgabe 22 erstreckenden Behandlungsstrecke bewegt und dabei durch das UV-Licht bzw. die UV-Strahlung der aktivierten UV-Lampen 17 und 18 entkeimt.

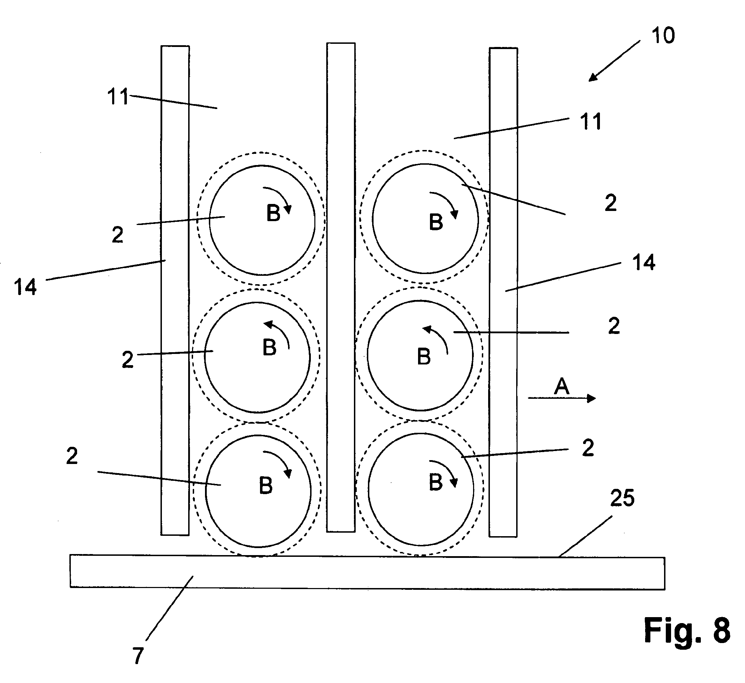

Wie in der Figur 8 dargestellt, wälzt sich bei der UV-Behandlung durch die mit dem Pfeil A angedeutete Drehbewegung des Rotors 10 der unterste in der jeweiligen Verschlussaufnahme 11 angeordnete Verschluss mit seiner Umfahgsfläche reibradartig auf einer von der unteren Gehäusewand 7 gebildeten und mit dem Rotor 10 nicht mitbewegten Verschlussanlagefläche 25 ab, wodurch entsprechend den Pfeilen B für die jeweils untersten Verschlüsse 2 eine Dreh- oder Abwälzbewegung erzeugt wird, die sich von Verschluss zu Verschluss auf sämtliche in der jeweiligen Verschlussaufnahme 11 angeordneten und mit ihren Umfangflächen gegeneinander anliegenden Verschlüsse 2 übertragt, sodass sich schließlich sämtliche Verschlüsse 2 in jeder Verschlussaufnahme 11 entsprechend den Pfeilen B gegenläufig drehen und dadurch an ihrer gesamten Oberfläche mit der UV-Strahlung beaufschlagt und optimal entkeimt bzw. sterilisiert werden.

Immer dann, wenn eine Verschlussaufnahme 11 des Rotors 10 die Verschlussabgabe 22 erreicht hat, fallen sämtliche, sterilisierten Verschlüsse 2 dieser Verschlussaufnahme in die Verschlussförderstrecke 23, über die die entkeimten bzw. sterilisierten Verschlüsse 2 dann der weiteren Verwendung zugeführt werden.

Die UV-Lampen 17 und 18 sind beispielsweise während des Betriebs der Vorrichtung 1 ständig aktiviert. Unter Berücksichtigung des Durchmessers des Rotors 10 ist die Geschwindigkeit der getakteten Drehbewegung des Rotors 10 so gewählt, dass die Dauer, die die die Verschlussaufnahme 11 für die Bewegung von der Verschlussaufgabe 20 bis an die Verschlussabgabe 22 benötigen, der für den angestrebten Entkeimungsgrad notwendigen Behandlungszeit, beispielsweise einer Behandlungszeit von 120 Sekunden entspricht.

Grundsätzlich besteht aber auch die Möglichkeit, die Ansteuerung der UV-Lampen 17 und 18 so auszuführen, dass diese insbesondere zur Steuerung der UV-Strahlungs-Leistung zu- und abschaltbar sind, d.h. getaktet oder impulsförmig angesteuert werden.

Um ein Eindringen von Keimen in den Innenraum 4 des Gehäuses 3 vermeiden, ist dieser Innenraum mit einem Überdruck, vorzugsweise mit dem Überdruck steriler Luft beaufschlagt. Die Entkeimung bzw. Sterilisation der Verschlüsse 2 erfolgt zum einen direkt durch die UV-Strahlung (UV-C-Strahlung), aber unterstützt auch durch das von dieser UV-Strahlung erzeugte Ozon, welches sich im Innenraum 4 sammelt.

Die besonderen Vorteile der Vorrichtung 1 bestehen darin, dass eine chemiefreie Sterilisation bzw. Entkeimung der Verschlüsse 2 erfolgt, dass die Vorrichtung 1 mit geringem Bauvolumen kompakt realisierbar ist, dass hohe Entkeimungsraten möglich sind, wobei die Entkeimungsleistung insbesondere auch durch die Geschwindigkeit der getakteten Drehbewegung des Rotors 10 und/oder durch die von den UV-Lampen 17 und 18 abgegebene UV-Leistung einstellbar ist, und dass durch die Eigendrehung der Verschlüsse 2 (Pfeile B) in den Verschlussaufnahmen 11 eine gleichbleibende reproduzierbare Qualität der Sterilisation bzw. Entkeimung erreicht wird. Ein weiterer wesentlicher Vorteil besteht in den reduzierten Betriebskosten der Vorrichtung 1, die insbesondere durch den relativ geringen Energieverbrauch für die UV-Lampen 17 und 18 sowie durch die relativ große Lebensdauer dieser Lampen (bis zu 15 000 Betriebsstunden) bedingt sind.

Zusätzlich zu den vorstehend beschriebenen Funktionselementen weist die Vorrichtung 1 beispielsweise auch Einrichtungen für eine Reinigung, insbesondere für eine Schaumreinigung der Vorrichtung, Einrichtungen für ein Absaugen von Ozon, Mess- und Überwachungsgeräte oder -einrichtungen, insbesondere auch zur Funktionsüberwachung der UV-Lampen 17 und 18 sowie Düsen für den Transport der Verschlüsse 2 durch Förderluft usw. auf.

Die Erfindung wurde voranstehend an einem Ausführungsbeispiel beschrieben. Es versteht sich, dass zahlreiche Änderungen sowie Abwandlungen möglich sind, ohne dass dadurch der der Erfindung zugrundeliegende Erfindungsgedanke verlassen wird.

Vorstehend wurde davon ausgegangen, dass das Sterilisieren bzw. Entkeimen der Verschlüsse 2 in einem einzigen Rotor 10 bzw. in den dortigen Verschlussaufnahmen 11 erfolgt. Grundsätzlich besteht aber die Möglichkeit, die Vorrichtung kaskaden- oder modulartig auszubilden, und zwar beispielsweise mit wenigstens zwei Rotoren 10, die dann mit ihren Rotorachsen z.B. achsgleich angeordnet sind, und zwar derart, dass die in einem ersten Rotor 10 behandelten Verschlüsse 2 an der Verschlussabgabe dieses Rotors nicht in die Verschlussförderstrecke 23 gelangen, sondern über eine Verschlussaufgabe in jeweils eine Verschlussaufnahme 11 des weiteren Rotors 10, mit dem die Verschlüsse 2 dann durch eine weitere Behandlungszone oder -strecke, beispielsweise eine weitere UV-Behandlungszone bewegt werden. Grundsätzlich besteht insbesondere bei einem derartigen kaskaden- oder modulartigen Aufbau auch die Möglichkeit, wenigstens zwei vom Behandlungsmedium her jeweils unterschiedliche Behandlungszonen vorzusehen.

### Bezugszeichenliste

- 1: Vorrichtung
- 2: Verschluss
- 3: Vorrichtungsgehäuse
- 4: Innenraum
- 5: Umfangswand
- 6: obere Gehäusewand
- 7: untere Gehäusewand
- 8: domartiger Abschnitt der oberen Gehäusewand
- 9: Gehäuseteil
- 10: Rotor
- 11: Verschlussaufnahme
- 12: Halterung
- 13: Tragerelement
- 14: Verschlussführungselement
- 15: Lager
- 16: Antrieb
- 17, 18: UV-Lampe, UV-Strahlungsquelle
- 19: Lampenfassung
- 20: Verschlussaufgabe
- 21: Verschlussförderstrecke
- 22: Verschlussabgabe
- 23: Verschlussförderstrecke
- 24: Verschlussführung
- 25: Fläche
- 26: Vertiefung
- A: Drehrichtung des Rotors 10
- B: Drehrichtung der Verschlüsse 2
- MA: Maschinenachse

## Patentansprüche

1. Vorrichtung zum Sterilisieren von Verschlüssen (2) zum Verschließen von Behältern mit einem Transportsystem zum Bewegen der Verschlüsse (2) durch wenigstens eine Behandlungszone (4), in der die Verschlüsse (2) für das Sterilisieren oder Entkeimen mit einer UV-Strahlung beaufschlagt werden, **dadurch gekennzeichnet, dass**
das Transportsystem in der wenigstens einen Behandlungszone (4) wenigstens einen um eine vertikale Maschinen- oder Rotorachse (MA) umlaufend antreibbaren Rotor (10) mit einer Vielzahl von am Umfang dieses Rotors (10) ausgebildeten Verschlussaufnahmen (11) aufweist, mit denen die Verschlüsse (2) vertikal von oben nach unten durch die wenigstens eine Behandlungszone (4) auf einer Behandlungsstrecke zwischen einer Verschluseaufgabe (20) und einer Verschlussabgabe (22) bewegt werden, wobei die Verschlussaufnahmen (11) mit ihrer Längserstreckung in Richtung der Rotorachse (MA) orientiert sind, und wobei an der wenigstens einen Behandlungsstrecke eine Vielzahl von UV-Licht aussendenden UV-Strahlungsquellen (17, 18) vorgesehen ist.

2. Vorrichtung nach Anspruch 1, **dadurch gekennzeichnet, dass** der wenigstens eine Rotor (10) hohltrommel- oder hohlzylinderartig mit einem von einer Vielzahl von Verschlussaufnahmen (11) gebildeten käfigartigen Trommelmantel ausgeführt ist, wobei die Verschlussaufnahmen (11) ihrerseits bevorzugt jeweils käfigartig mit sich in Längsrichtung der jeweiligen Verschlussaufnahme (11) erstreckenden stab- und/oder stangenförmigen Verschlussführungsschlenen (14) ausgebildet sind, zwischen denen die Verschlüsse (2) in der jeweiligen Verschlussaufnahme (11) aufgenommen sind.

3. Vorrichtung nach einem der vorhergehenden Anspruche, **dadurch gekennzeichnet, dass** die UV-Strahlungsquellen (17, 18) eine Längserstreckung parallel zur Rotorachse (MA) aufweisen.

4. Vorrichtung nach Anspruch 4, **dadurch gekennzeichnet, dass** die UV-Strahlungsquellen jeweils von mehreren in Längsrichtung der jeweiligen UV-Strahlungsquelle (17, 18) gegeneinander versetzten stabförmigen UV-Strahlern oder stabförmigen UV-Lampen (17,18) gebildet sind.

5. Vorrichtung nach einem der vorhergehenden Ansprüche, **dadurch gekennzeichnet, dass** die UV-Strahlungsquellen (17, 18) an der Bewegungsbahn der Verschlussaufnahmen (11) innenliegend und/oder außenliegend mit diesen Verschlussaufnahmen (11) oder mit dem Rotor (10) nicht umlaufend vorgesehen sind.

6. Vorrichtung nach einem der vorhergehenden Ansprüche, **dadurch gekennzeichnet, dass** die UV-Strahlungsquellen (17, 18) an einer den Innenraum der Vorrichtung (1) an der Oberseite abschließenden Gehäusewand (6) hängend angeordnet sind.

7. Vorrichtung nach einem der vorhergehenden Ansprüche, **dadurch gekennzeichnet, dass** innere UV-Strahlungsquellen (18) an der Innenseite der Bewegungsbahn der Verschlussaufnahmen (11) gegenüber äußeren UV-Strahlungsquellen (17) an der Außenseite der Bewegungsbahn der Verschlussaufnahmen (11) versetzt sind, vorzugsweise derart, dass jede innere UV-Strahlungsquelle (18) bezogen auf die Rotorachse (MA) radial einer Lücke zwischen zwei äußeren UV-Strahiungsquellen (17) gegenüberliegt.

8. Vorrichtung nach einem der vorhergehenden Ansprüche, **dadurch gekennzeichnet, dass** die Behandlungszone (4) derart ausgebildet ist, um mit dem Überdruck eines sterilen gas- und/oder dampfförmigen Mediums, vorzugsweise mit dem Überdruck steriler Luft, beaufschlagbar zu sein.

9. Vorrichtung nach einem der vorhergehenden Ansprüche, **dadurch gekennzeichnet, dass** einem unteren, offen Ende der Verschlussaufnahmen (11) gegenüberliegend wenigstens eine mit dem Rotor (10) nicht mitbewegte oder relativ zum Rotor (10) bewegte Verschlussanlageflache (25) vorgesehen ist, auf der sich der dieser Verschlussanlagefläche (25) benachbarte Verschluss (2) bei umlaufendem Rotor (10) zur Erzeugung einer von Verschluss zu Verschluss übertragenen Verschluss-Drehbewegung (B) in der Verschlussaufnahme (11) abwälzt.

10. Vorrichtung nach einem der vorhergehenden Ansprüche, **dadurch gekennzeichnet, dass** das Transportsystem mehrere Transportrichtung der Verschlüsse (2) aufeinander folgende und jeweils in wenigstens einer Behandlungszone angeordnete Rotoren (10) mit jeweils einer Vielzahl von Verschlussaufnahmen (11) aufweist.

## Claims

1. A device for sterilising closures (2) for closing containers, comprising a conveying system for moving the closures (2) through at least one treatment zone (4) in which the closures (2) are exposed to UV radiation for sterilisation or disinfection, wherein the conveying system in the at least one treatment zone (4) exhibits a rotor (10) which can be driven to rotate about a vertical machine axis or rotor axis (MA) and which has a plurality of closure recipients (11) configured on the periphery of this rotor (10) and with which the closures (2) are moved vertically from an upper to a lower position through the at least one treatment zone (4) on a treatment section between a closure feeder (20) and a closure dispenser (22), the closure recipients (11) being oriented with their longitudinal extension in the direction of the rotor axis and a plurality of UV-light-emitting UV radiation sources (17, 18) being provided on the at least one treatment section.

2. The device of claim 1 wherein the at least one rotor (10) is conformed in the manner of a hollow drum or hollow cylinder having a cage-like drum jacket formed by a plurality of closure recipients (11), with the closure recipients (11) in turn being configured preferably each in the manner of a cage with rod-like and/or bar-like closure guide rails (14) extending in the longitudinal direction of the respective closure recipient (11), between which the closures (2) are received in the respective closure recipient (11).

3. The device of any one of the preceding claims wherein the UV radiation sources (17, 18) exhibit a longitudinal extension parallel to the rotor axis (MA).

4. The device of claim 4 wherein each UV radiation source is formed by a plurality of rod-like UV radiators or rod-like UV lamps (17, 18) which are offset relative to one another in the longitudinal direction of the respective UV radiation source (17,18).

5. The device of any one of the preceding claims wherein the UV radiation sources (17, 18) are provided lying on the inside and/or outside along the trajectory of the closure recipients (11) but not circulating with those closure recipients (11) or with the rotor (10).

6. The device of any one of the preceding claims wherein the UV radiation sources (17, 18) are disposed suspended on a housing wall (6) which closes the interior of the device (1) at its upper side.

7. The device of any one of the preceding claims wherein inner UV radiation sources (18) on the inside of the trajectory of the closure recipients (11) are staggered opposite outer UV radiation sources (17) on the outside of the trajectory of the closure recipients (11), preferably in such a way that relative to the rotor axis (MA) each inner UV radiation source (18) lies radially opposite a gap between two outer UV radiation sources (17).

8. The device of any one of the preceding claims wherein the treatment zone (4) is configured so that it can be exposed to the positive pressure of a sterile gaseous and/or vaporous medium, preferably to the positive pressure of sterile air.

9. The device of any one of the preceding claims wherein there is provided lying opposite a lower open end of the closure recipients (11) at least one closure abutment face (25) which is not moved with the rotor (10) or which is moved relative to the rotor (10) and on which, as the rotor (10) rotates, the closure (2) neighbouring this closure abutment face (25) moves on rolling contact in the closure recipient (11) so as to generate a closure rotary motion (B) that is transmitted from closure to closure.

10. The device of any one of the preceding claims wherein the conveying system exhibits a plurality of rotors (10) following one another in the direction of transport of the closures (2) and each arranged in at least one treatment zone and each having a plurality of closure recipients (11).

## Revendications

1. Dispositif servant à stériliser des fermetures (2) servant à fermer des contenants, comprenant un système de transport servant à déplacer les fermetures (2) à travers au moins une zone de traitement (4), dans laquelle les fermetures (2) sont exposées à un rayonnement UV aux fins de leur stérilisation ou de leur désinfection, **caractérisé en ce**
**que** le système de transport présente, dans la zone de traitement (4) au moins au nombre de une, au moins un rotor (10) pouvant être entraîné en rotation autour d'un axe de rotor (MA) ou d'un axe de machine vertical et comprenant une pluralité de logements de fermeture (11) réalisés sur la périphérie dudit rotor (10), lesquels logement permettent de déplacer les fermetures (2) verticalement depuis le haut vers le bas à travers la zone de traitement (4) au moins au nombre de une, sur une ligne de traitement entre un système de dépôt de fermeture (20) et un système de retrait de fermeture (22), sachant que les logements de fermeture (11) sont orientés, par leur extension longitudinale, en direction de l'axe de rotor (MA), et sachant qu'une pluralité de sources de rayonnement UV (17, 18) émettant de la lumière UV est prévue au niveau de la ligne de traitement au moins au nombre de une.

2. Dispositif selon la revendication 1, **caractérisé en ce que** le rotor (10) au moins au nombre de un est réalisé à la manière d'un tambour creux ou d'un cylindre creux avec une gaine de tambour semblable à une cage formée par une pluralité de logements de fermeture (11), sachant que les logements de fermeture (11) quant à eux sont réalisés de préférence respectivement de manière semblable à une cage avec des rails de guidage de fermeture (14) en forme de barre et/ou de tige s'étendant dans la direction longitudinale du logement de fermeture (11) respectif, les fermetures (2) étant logées, dans le logement de fermeture (11) respectif, entre lesdits rails de guidage de fermeture.

3. Dispositif selon l'une quelconque des revendications précédentes, **caractérisé en ce que** les sources de rayonnement UV (17, 18) présentent de manière parallèle à l'axe de rotor (MA) une extension longitudinale.

4. Dispositif selon la revendication 4, **caractérisé en ce que** les sources de rayonnement UV sont formées respectivement par plusieurs projecteurs à UV ou lampes à UV (17, 18) en forme de barre décalés les uns par rapport aux autres dans la direction longitudinale de la source de rayonnement UV (17, 18) respective.

5. Dispositif selon l'une quelconque des revendications précédentes, **caractérisé en ce que** les sources de rayonnement UV (17, 18) sont prévues, de manière à se trouver côté intérieur et/ou côté extérieur au niveau de la trajectoire de déplacement des logements de fermeture (11), sans être amenées en rotation avec lesdits logements de fermeture (11) ou avec le rotor (10).

6. Dispositif selon l'une quelconque des revendications précédentes, **caractérisé en ce que** les sources de rayonnement UV (17, 18) sont attachées au niveau d'une paroi de boîtier (6) fermant l'espace intérieur du dispositif (1) au niveau du côté supérieur.

7. Dispositif selon l'une quelconque des revendications précédentes, **caractérisé en ce que** des sources de rayonnement UV intérieures (18) sont décalées, au niveau du côté intérieur de la trajectoire de déplacement des logements de fermeture (11), par rapport aux sources de rayonnement UV extérieures (17) au niveau du côté extérieur de la trajectoire de déplacement des logements de fermeture (11), de préférence de telle manière que chaque source de rayonnement UV intérieure (18) fait face, par rapport à l'axe de rotor (MA), radialement à un espace entre deux sources de rayonnement UV extérieures (17).

8. Dispositif selon l'une quelconque des revendications précédentes, **caractérisé en ce que** la zone de traitement (4) est réalisée de manière à pouvoir être exposée à la surpression d'un milieu stérile sous forme de gaz et/ou sous forme de vapeur, de préférence à la surpression de l'air stérile.

9. Dispositif selon l'une quelconque des revendications précédentes, **caractérisé en ce qu'**est prévue de manière à faire face à une extrémité inférieure ouverte des logements de fermeture (11), au moins une surface d'appui de fermeture (25), qui n'est pas entraînée avec le rotor (10) ou qui est déplacée par rapport au rotor (10), sur laquelle la fermeture (2) contigüe à ladite surface d'appui de fermeture (25) roule, lorsque le rotor (10) tourne, pour produire un mouvement de rotation de fermeture (B) transmis de fermeture en fermeture, dans le logement de fermeture (11).

10. Dispositif selon l'une quelconque des revendications précédentes, **caractérisé en ce que** le système de transport présente plusieurs rotors (10) suivant consécutivement la direction de transport des fermetures (2) et disposés respectivement dans au moins une zone de traitement, comprenant respectivement une pluralité de logement de fermeture (11).
